# EUROPÄISCHE PATENTANMELDUNG

(11) **EP 3 232 152 A1**
(43) Veröffentlichungstag der Anmeldung: **18.10.2017**
(21) Anmeldenummer: 17166201.8
(22) Anmeldetag: 12.04.2017
(51) Int. Cl.: G01B 11/24, G01N 21/89

(54) **LASERSCANVORRICHTUNG ZUR OPTISCHEN QUALITÄTSBEURTEILUNG UND VERMESSUNG VON OBJEKTEN IM QUERTRANSPORT**

(30) Priorität: 15.04.2016 DE 102016106994
(71) Anmelder: ATB Blank GmbH, 89297 Roggenburg (DE)
(72) Erfinder: BLANK, Georg, 89297 Roggenburg (DE)
(74) Vertreter: Baur & Weber Patentanwälte PartG mbB

(57) **Zusammenfassung**

Es wird eine Laserscanvorrichtung zur optischen Qualitätsbeurteilung und Vermessung von Objekten (OB) im Quertransport, insbesondere von Schnitthölzern, beschrieben, wobei die Laserscanvorrichtung (VO) zur Triangulationsmessung eine Lichtquelle (LQ) zur Abgabe eines Laserstrahls (ST1) in Richtung des Objekts (OB) und einen Lichtsensor (LS) zur Aufnahme der von dem Objekt (OB) reflektierten Laserstrahl (ST2) umfasst, wobei die Lichtquelle (LQ) und der Lichtsensor (LS) relativ zu dem Objekt (OB) korreliert und in gleicher Drehrichtung schwenkbar sind, so dass die Lichtquelle (LQ) zusammen mit dem Lichtsensor (LS) das Objekt (OB) entlang einer Längsachse des Objekts (OB) abscannt, um ein dreidimensionales Abbild der Oberfläche des Objekts (OB) zu erzeugen.

## Beschreibung

Die Erfindung betrifft eine Laserscanvorrichtung zur optischen Qualitätsbeurteilung und Vermessung von Objekten im Quertransport, insbesondere von Schnitthölzern.

Aus dem allgemeinen Stand der Technik ist es bekannt, dass Schnitthölzer hauptsächlich in Sägewerken und Hobelwerken aber auch in der Möbel- oder Parkettproduktion mittels optischer Messverfahren kontrolliert werden, um anhand eines dreidimensionalen Abbildes die Qualität der Holzoberflächen, eine eventuelle Schüsselung oder andere qualitätslimitierende Eigenschaften bereits im Produktionsprozess quantitativ und qualitativ bestimmen zu können. Derartige Schnitthölzer, üblicherweise in der Form eines Bretts, werden dabei oftmals in Längsrichtung mittels eines Laserscanners optisch vermessen.

Ein Beispiel für eine Scannvorrichtung in Längsrichtung wird in der DE 602 16 623 T2 beschrieben. In dieser Druckschrift wird eine Anordnung zum Abbilden der Merkmale eines Gegenstands mittels eines Messsystems beschrieben, bei welchem das Messsystem und/oder der Gegenstand relativ zueinander in einer vorgegebenen Bewegungsrichtung bewegt werden, wobei der Gegenstand vorzugsweise relativ zu dem Messsystem bewegt wird. Der Gegenstand wird mit einfallendem Licht beleuchtet, das in der Bewegungsrichtung eine eingeschränkte Ausdehnung hat, und von dem Gegenstand reflektiertes Licht wird mittels eines Bildsensors erfasst, der auf derselben Seite des Gegenstands wie das einfallende Licht angeordnet ist, wobei der Bildsensor das erfasste Licht in elektrische Ladungen umwandelt, gemäß welchen eine digitale Darstellung der Merkmale des Gegenstands erzeugt wird.

Ein Verfahren zur kontinuierlichen optoelektronischen Aufnahme von Eigenschaften des Holzes und Bestimmung der Holzqualität ist auch aus der DE 195 47 260 A1 bekannt. Dazu wird eine Kopplung von Messsystemen zur fotometrisch hochauflösenden Intensitätsmessung der Remission der Holzoberfläche mit Verfahren zur zweidimensional geometrisch hochauflösenden Intensitätsmessung der Remission der Holzoberfläche und/oder zur dreidimensional geometrisch hochauflösenden Intensitätsmessung der Remission der Holzoberfläche vorgenommen. Die erhaltenen Messwerte werden zum einen in physikalische Farbsysteme, welche der Helligkeit- und Farbwahrnehmung des Menschen nachempfunden sind und zum anderen in geometrisch exakte Bilder liefernde Systeme umgewandelt.

Ein Nachteil der optischen Vermessung in Längsrichtung besteht jedoch darin, dass die Korrelation zwischen einzelnen Bildpunkten und dem zu untersuchenden Objekt mitunter schwierig ist, da ein derartiges Laserscansystem keine Absolutkoordinaten auf dem Objekt liefert. Dazu wird üblicherweise die Information des Laserscanners mit einer Messeinrichtung zur Bestimmung der Längsrichtung verknüpft.

Oftmals wird jedoch beispielsweise in einem Sägewerk das zu verarbeitende Objekt nicht in Längsrichtung sondern in Querrichtung transportiert. Um in diesem Fall eine ausreichende Genauigkeit bei der optischen Vermessung erreichen zu können ist es daher üblich, mehrere optische Vermessungssysteme in Längsrichtung des Objekts nebeneinander liegend anzuordnen. Die einzelnen Messsysteme überdecken dabei nur einen kleinen Teil der Oberfläche des Objekts, so dass deren Bilder anschließend zu einem Gesamtabbild zusammen gefügt werden müssen.

Ein Beispiel für ein derartiges Verfahren bzw. Vorrichtung zum Vermessen von Schnitthölzern im Querdurchlauf ist aus der DE 42 32 529 A1 bekannt. Die Vermessung erfolgt mittels mehrerer Paare von Lichtsendern, die entlang des Holzes verteilt sind und deren Lichtsender das Holz von entgegengesetzten Seiten einer Messlinie her schräg auf die Breitseite gerichtet beleuchten. Jedem Lichtsenderpaar ist ein Lichtempfänger zugeordnet, der nur Licht empfängt, das in einem definierten, begrenzten Empfangsbereich von der Holzoberfläche her reflektiert wird. Die Lichtempfänger erzeugen bei einem Intensitätssprung im empfangenen Licht ein elektrisches Signal.

Bekannte Systeme zur Vermessung von Schnitthölzern im Querdurchlauf benötigen daher einen erhöhten Installationsaufwand, der durch die Vielzahl von Lichtsendern und Empfängern hervorgerufen wird. Daher sind derartige Systeme sehr aufwändig und in der industriellen Fertigung mit hohen Kosten verbunden.

Es besteht daher in der Technik ein Bedarf, eine entsprechende Vorrichtung zu entwickeln, die eine kosteneffiziente Vermessung von Schnitthölzern im Querdurchlauf ermöglicht.

Es ist daher Aufgabe der Erfindung, eine Laserscanvorrichtung zur optischen Vermessung von Objekten anzugeben, die die im Quertransport auftretenden Probleme überwindet, indem eine kostengünstige Realisierung ermöglicht wird.

Diese Aufgabe wird durch die Merkmale des unabhängigen Patentanspruchs 1 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.

Gemäß der Erfindung wird eine Laserscanvorrichtung zur optischen Qualitätsbeurteilung und Vermessung von Objekten im Quertransport, insbesondere von Schnitthölzern, wobei die Laserscanvorrichtung zur Triangulationsmessung eine Lichtquelle zur Abgabe eines Laserstrahls in Richtung des Objekts und einen Lichtsensor zur Aufnahme der von dem Objekt reflektierten Laserstrahl umfasst, wobei die Lichtquelle und der Lichtsensor relativ zu dem Objekt korreliert und in gleicher Drehrichtung schwenkbar sind, so dass die Lichtquelle zusammen mit dem Lichtsensor das Objekt entlang einer Längsachse des Objekts abscannt, um ein drei-dimensionales Abbild der Oberfläche des Objekts zu erzeugen.

Demnach wird die im Stand der Technik angegebene mehrfache Bereitstellung von Lichtquellen und -sensoren vermieden, so dass mit einer einzigen Lichtquelle und einem einzigen Lichtsensor ein Abscannen der Oberfläche des Objekts möglich ist. Dies wird dadurch erreicht, dass die Lichtquelle und der Lichtsensor korreliert geschwenkt werden, wobei die Schwenkbewegung dabei in einer Ebene ausgerichtet ist, die parallel zu der zu vermessenden Oberfläche des Objekts liegt. Demnach kann durch das Schwenken mittels der Lichtquelle die komplette Oberfläche des Objekts ausgeleuchtet werden, wobei der Lichtsensor entsprechend mit bewegt wird, so dass das reflektierte Licht während des Schwenkvorgangs auf den Lichtsensor trifft. Demnach wird eine effiziente und kostengünstige Lösung zur Vermessung von Objekten im Quertransport geschaffen.

Gemäß einer Ausführungsform der Erfindung sind die Lichtquelle und der Lichtsensor jeweils um eine Drehachse schwenkbar. Die Lichtquelle und der Lichtsensor können aber auch auf gegenüberliegenden Enden eines gemeinsamen Schwenkarms montiert sein, der um eine Drehachse schwenkbar ist.

Demnach kann die korrelierte Bewegung der Lichtquelle und des Lichtsensors zur Triangulationsmessung entweder auf einem einzelnen Schwenkarm um eine Drehachse oder über zwei getrennte Drehachsen erfolgen. Zur Triangulationsmessung werden in beiden Fällen die aufgenommenen Bilder des Lichtsensors mit den Drehwinkeln korreliert.

Gemäß einer weiteren Ausführungsform der Erfindung ist die Lichtquelle eine Laserdiode und/oder der Lichtsensor eine CCD-Kamera.

Derartige Ausgestaltungen stellen fachübliche Komponenten eines Laserscanners dar, so dass die Erfindung mit handelsüblichen Bauteilen realisierbar ist.

Gemäß einer weiteren Ausführungsform der Erfindung sind die von dem Lichtsensor abgegeben Signale zur Erzeugung des dreidimensionalen Abbilds der Oberfläche des Objekts während des Schwenkens mit dem jeweiligen Drehwinkel korreliert.

Zur Erzeugung eines dreidimensionalen Abbilds kann eine Absolutposition auf dem Objekt durch entsprechende Verknüpfung der Signale des Lichtsensors an einer bestimmten Messposition mit dem bei der Aufnahme des Abbilds vorliegenden Drehwinkel erfolgen. Demnach ist eine dreidimensionale Rekonstruktion des Abbildes der Oberfläche des Objekts durch die bekannte Lage des Schwenkarms, sowie dem Abstand des Lichtsensors bzw. der Lichtquelle zur Drehachse und dem Abstand zum Objekt gegeben.

Gemäß einer weiteren Ausführungsform der Erfindung erfolgt das Schwenken mit einer einstellbaren Geschwindigkeit.

Durch die Wahl der Geschwindigkeit des Schwenkens des Schwenkarms kann zum einen die Dauer eines kompletten Scanvorgangs über das Objekt beeinflusst werden und zum anderen die Auflösung der Laserscanvorrichtung entsprechend justiert werden. Bei hohen Drehgeschwindigkeiten wird üblicherweise die abzugebende Datenrate des Lichtsensors erhöht, um auch bei hoher Schwenkgeschwindigkeit eine gute Auflösung zu erreichen. Bei niedrigen Drehgeschwindigkeiten kann der Lichtsensor an sich mit höherer Datenrate betrieben werden, da aufgrund der längeren Zeitdauer eine größere Datenmenge ausgelesen werden kann. Dies geht mit einer höheren Auflösung einher.

Gemäß einer weiteren Ausführungsform der Erfindung ist die Intensität des von der Lichtquelle abgegebenen Lichtstrahls in Abhängigkeit des Drehwinkels gesteuert.

Demnach können Intensitätsunterschiede auf dem Objekt verringert werden, da durch das Schwenken des Schwenkarms sich der Abstand zwischen Laserscanvorrichtung und dem zu messenden Objekt in Abhängigkeit des Drehwinkels verändert. Sofern der Laserscanner mittig über dem Objekt angeordnet ist, würde bei einem Schwenken zu den jeweiligen Enden des Objekts der Abstand vergrößert sein, so dass dort die Lichtquelle aus der Mittenlage mit größerer Intensität beim Schwenken zu den Enden hin betrieben werden kann. Demnach ist die Intensität des Laserstrahls über die gesamte Oberfläche des Objekts konstant oder nahezu konstant.

Gemäß einer weiteren Ausführungsform ist die Empfindlichkeit des Lichtsensors von der Drehposition in Abhängigkeit des Drehwinkels parametrisiert.

Ähnlich wie im vorherigen Abschnitt bezüglich des variablen Abstands zwischen Laserscanvorrichtung und Objekt bereits beschrieben, kann auch die Empfindlichkeit des Lichtsensors entsprechend der Drehposition parametrisiert sein, um die damit verbundenen veränderten Abbildungseigenschaften kompensieren zu können.

Gemäß einer weiteren Ausführungsform der Erfindung erfolgt die optische Vermessung des Objekts im Stillstand.

Insbesondere in Sägewerken sind an bestimmten Stellen des Verarbeitungsprozesses Schnitthölzer wie Bretter oder dergleichen nach Ende eines Bearbeitungsschritts kurzfristig im Stillstand, wobei dort eine Kante des Objekts typischerweise an einer Führungsschiene oder dergleichen anliegt. Während dieser Phase kann nun eine Vermessung der Oberfläche des Objekts durch die Laserscanvorrichtung erfolgen. Dabei kann je nach Zeitdauer des Stillstands die Auflösung, wie bereits oben beschrieben, entsprechend angepasst werden.

In einer weiteren Ausführungsform der Erfindung erfolgt die optische Vermessung des Objekts im Durchlauf.

Dabei können das Sichtfeld des Lichtsensors und/oder die Beleuchtungsfläche der Lichtquelle an die Durchlaufgeschwindigkeit des Objekts angepasst sein. In einer anderen Variante kann eine Anpassung an die Durchlaufgeschwindigkeit durch eine Verkippung um eine senkrecht zur Drehachse und der Durchlaufrichtung angeordnete Stellachse erfolgen, wobei die Verkippung an die Durchlaufgeschwindigkeit angepasst ist.

Demnach werden zwei Lösungsmöglichkeiten für die optische Vermessung des Objekts im Durchlauf vorgeschlagen. Bei der ersten Variante wird das Sichtfeld der Laserscanvorrichtung so groß gewählt, dass bei einem Abscannen der Oberfläche des Objekts entlang einer Längsrichtung das Objekt während des Weiterbewegens entlang der Durchlaufrichtung das Messfenster nicht verlässt. Bei der anderen Variante wird die Laserscanvorrichtung senkrecht zur Drehachse und zur Durchlaufrichtung gekippt, so dass der von der Lichtquelle abgegebene Laserstrahl der kontinuierlichen Weiterführung des Objekts folgt. Die Verkippung wird dabei an die Durchlaufgeschwindigkeit angepasst, wobei dies beispielsweise mittels eines Stellmotors im laufenden Betrieb geschehen kann. Bei zunehmender Durchlaufgeschwindigkeit muss die Laserscanvorrichtung entsprechend stärker gekippt werden. Vorteilhafterweise erfolgt dabei das Schwenken um die Drehachse von einem ersten Ende zu einem zweiten Ende des Objekts hin, wobei bei Erreichen des zweiten Endes ein schnelles Zurückschwenken an das erste Ende erfolgt. Dies ermöglicht eine einfache und präzise Rekonstruktion des dreidimensionalen Abbilds der Oberfläche des Objekts.

Gemäß einer weiteren Ausführungsform der Erfindung erfolgt die optische Vermessung des Objekts auf einer Auflage, die eine Kippeinrichtung des Objekts aufweist, um mehrere Seitenflächen des Objekts vermessen zu können.

Demnach ist es möglich, in einem ersten Schritt eine Vorderseite des Objekts zu vermessen und nach Kippen des Objekts in einem zweiten Schritt die Rückseite anschließend ebenfalls optisch zu vermessen. In weiteren Ausführungen wäre es ebenfalls denkbar, auch die übrigen Seitenflächen entsprechend optisch zu vermessen.

Dabei ist mittels der Laserscanvorrichtung auch eine Vermessung von gleichzeitig zwei Seiten des Objekts möglich, in dem das Objekt OB entweder schräg zur Laserscanvorrichtung angeordnet ist oder ein Schrägstellen des Objekts erfolgt. Demnach kann beispielsweise ein Brett gleichzeitig an seiner Hauptfläche und seiner schmäleren Seitenfläche vermessen werden.

Gemäß einer weiteren Ausführungsform der Erfindung sind zusätzliche lichtemittierende Strahler, vorzugsweise benachbart zur Lichtquelle (oder benachbart zum Lichtsensor, angebracht, die das Objekt farbig und/oder punktförmig ausleuchten.

Der oder die zusätzlichen Strahler ermöglichen eine funktionale Erweiterung der Laserscanvorrichtung von der Vermessung zur Qualitätsbeurteilung. Dabei können ein oder zwei Strahler das Objekt farbig ausleuchten. Die farbige Ausleuchtung kann zur optischen Qualitätsbeurteilung der Oberfläche des Objekts herangezogen werden. Alternativ oder zusätzlich kann ein Punktlaser als zusätzlicher oder weiterer zusätzlicher lichtemittierender Strahler angebracht sein. Mittels eines Punktlasers lassen sich Faserungen oder Astlöcher optisch erkennen, so dass auch eine Beurteilung bezüglich Festigkeiten oder dergleichen möglich ist.

Gemäß einer weiteren Ausführungsform der Erfindung ist der Lichtsensor aus einer Mehrfachanordnung von lichtempfindlichen Elementen gebildet, wobei ein für eine Auswertung relevanter Bereich der Mehrfachanordnung in Abhängigkeit der Schwenkbewegung gewählt ist.

Während der Schwenkbewegung wandert ein Fokusbereich auf dem Lichtsensor, da die Scheimpflugbedingung nicht über die gesamte Fläche des Lichtsensors gleichzeitig erfüllt sein kann. Somit wird nicht der volle Sensorbereich verwendet, sondern für eine Auswertung ist nur ein kleinerer Bereich relevant. In Abhängigkeit der Schwenkbewegung wird daher nur derjenige Bereich ausgewählt, auf dem gerade gemessen werden soll. Dadurch erst ist es wählt, auf dem gerade gemessen werden soll. Dadurch erst ist es möglich, mehrere tausend Messungen pro Sekunde zu realisieren, da die Auswahl auf bestimmte Bereiche des Lichtsensors eine enorme Datenreduktion bewirkt.

Nachfolgend werden einige Ausführungsbeispiele anhand der Zeichnung näher erläutert. Es zeigen:
- Fig. 1: in einer schematischen Seitenansicht eine erfindungsgemäße Laserscanvorrichtung zusammen mit einem Objekt gemäß einer ersten Ausführungsform,
- Fig. 2: die Laserscanvorrichtung aus Fig. 1 bei Durchführen einer Schwenkbewegung in eine erste Richtung,
- Fig. 3: die Laserscanvorrichtung aus Fig. 1 bei Schwenken in eine zweite Richtung,
- Fig. 4: die Laserscanvorrichtung aus Fig. 1 in einer weiteren Seitenansicht,
- Fig. 5: in einer schematischen Seitenansicht eine erfindungsgemäße Laserscanvorrichtung zusammen mit einem Objekt gemäß einer zweiten Ausführungsform,
- Fig. 6: in einer schematischen Seitenansicht eine erfindungsgemäße Laserscanvorrichtung zusammen mit einem Objekt gemäß einer ersten Ausführungsform,
- Fig. 7: eine Draufsicht auf eine Auflage beim Vermessen des Messobjekts mit einer erfindungsgemäßen Laserscanvorrichtung, und
- Fig. 8: eine weitere Draufsicht beim Vermessen eines Objekts mit einer erfindungsgemäßen Laserscanvorrichtung.

In den Figuren sind gleiche oder funktional gleich wirkende Bauteile mit den gleichen Bezugszeichen versehen.

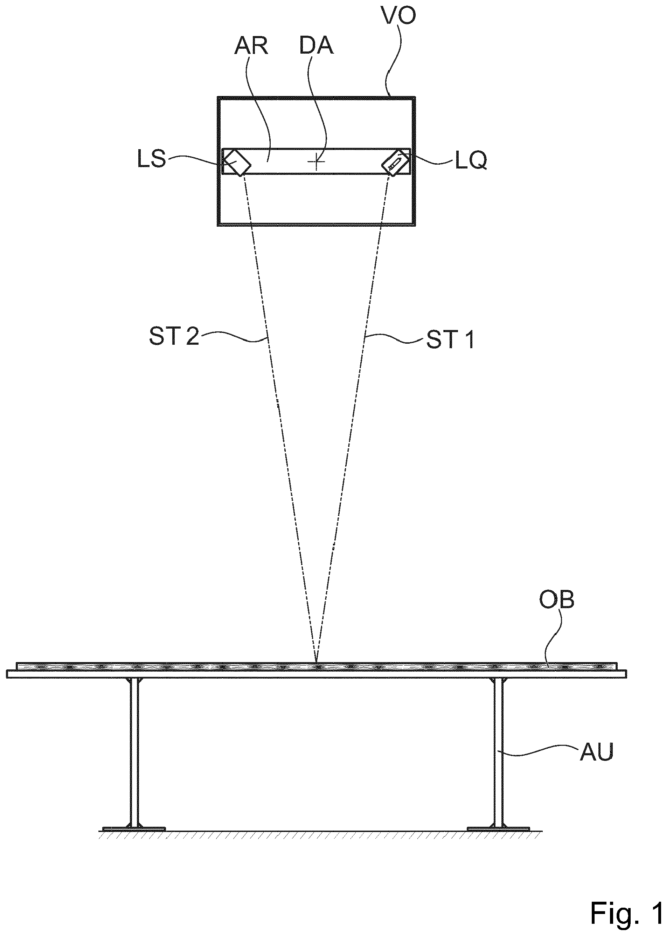

In Fig. 1 ist in einer Seitenansicht eine Laserscanvorrichtung VO gezeigt, die in etwa mittig über einem Objekt OB angeordnet ist. Die Seitenansicht der Fig. 1 zeigt das Objekt OB auf einer Auflage AU, wobei in der industriellen Fertigung typischerweise eine Vielzahl von Objekten OB bereitgestellt werden, die nacheinander mittels der Laserscanvorrichtung VO optisch vermessen werden sollen. Die Zuführung von Objekten OB erfolgt dabei im Quertransport, d. h. das in Fig. 1 gezeigte Objekt OB wird der Aufnahme AU in einer Ebene senkrecht zur Zeichenebene zu- bzw. abgeführt.

Die Laserscanvorrichtung VO umfasst eine Lichtquelle LQ in Form einer Laserdiode, die einen Laserstrahl ST1 in Richtung des Objekts OB abgeben kann. Desweiteren umfasst die Laserscanvorrichtung VO einen Lichtsensor LS, der den von dem Objekt OB reflektierten Laserstrahl ST2 aufnehmen kann. Die Lichtquelle LQ und der Lichtsensor LS sind auf gegenüber liegenden Enden eines Schwenkarms AR angeordnet. Zum Schwenken des Schwenkarms AR ist eine Drehachse DA vorgesehen, die im Wesentlichen senkrecht zur Zeichenebene der Fig. 1 angeordnet ist. Demnach kann durch Schwenken des Schwenkarms AR die Lichtquelle LQ mittels des Laserstrahls ST1 jeden Punkt auf der Oberfläche des Objekts OB erreichen.

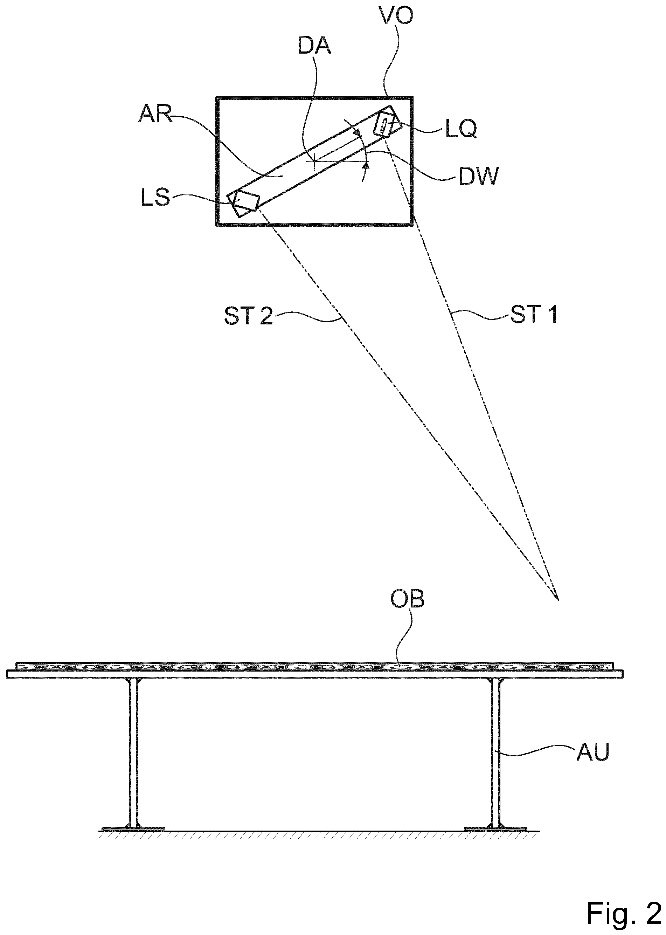

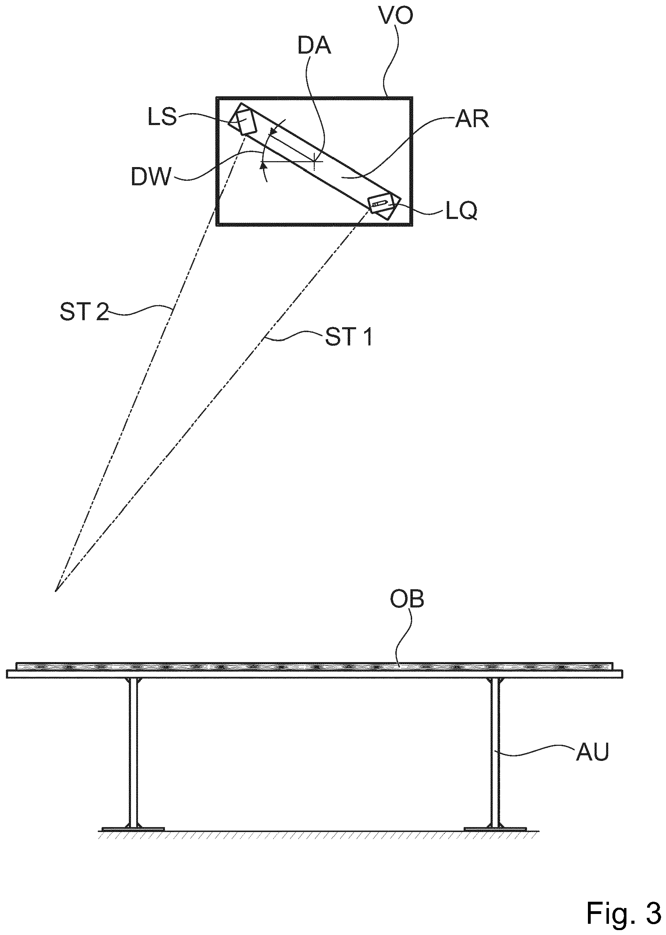

In Fig. 2 und Fig. 3 ist die Laserscanvorrichtung VO aus Fig. 1 nochmals gezeigt, wobei in Fig. 2 der Schwenkarm AR zu einem ersten Ende des Objekts OB ausgerichtet ist und in Fig. 3 entsprechend zu einem zweiten Ende des Objekts OB. Da sich aufgrund des Schwenkens des Schwenkarms AR der Abstand zwischen der Lichtquelle LQ bzw. dem Lichtsensor LS und der Oberfläche des Objekts OB kontinuierlich ändert, ist insbesondere für die Erfassung des reflektierten Lichts des Strahls ST2 eine hohe Tiefenschärfe in einem Objektiv des Lichtsensors LS notwendig. Dies kann beispielsweise dadurch erreicht werden, dass das Objektiv und der eigentliche Sensor des Lichtsensors LS unter einem bestimmten Winkel angeordnet sind, was üblicherweise als Scheimpflug-Objektiv in der Optik bekannt ist. Bei der Aufnahme eines Abbilds des Objekts OB ist es vorgesehen, die Lichtquelle während des Schwenkens des Schwenkarms AR um die Drehachse DA entsprechend in Abhängigkeit des Drehwinkels DW zu verändern, um eine homogene Beleuchtungsintensität auf der Oberfläche des Objekts OB erreichen zu können.

Ebenfalls ist es vorgesehen, dass der Lichtsensor LS in Abhängigkeit des Drehwinkels DW mit unterschiedlicher Belichtungszeit arbeitet, so dass der sich verändernde Abstand zwischen Lichtquelle LQ bzw. Lichtsensor LS und der Oberfläche des Objekts OB kompensiert werden kann. Die vom Lichtsensor LS aufgenommenen Daten werden dabei mit dem Drehwinkel DW korreliert, so dass aus der bekannten geometrischen Ausrichtung der Lichtquelle LQ bzw. des Lichtsensors LS auf dem Schwenkarm AR ein dreidimensionales Bild der Oberfläche des Objekts OB rekonstruiert werden kann. Der Lichtsensor LS ist typischerweise aus einem zweidimensionalen Array von lichtempfindlichen Elementen gebildet. Ein für eine Auswertung relevanter Bereich wird in Abhängigkeit der Schwenkbewegung gewählt, denn während der Schwenkbewegung wandert ein Fokusbereich auf dem Lichtsensor LS, da die Scheimpflugbedingung nicht über die gesamte Fläche des Lichtsensors LS gleichzeitig erfüllt sein kann. Somit wird nicht der volle Bereich des Lichtsensors LS verwendet. Dadurch erst ist es möglich, mehrere tausend Messungen pro Sekunde zu realisieren, da die Auswahl auf bestimmte Bereiche des Lichtsensors LS eine enorme Datenreduktion bewirkt.

Das Scannen der Oberfläche des Objekts OB kann dabei auf unterschiedliche Weise erfolgen, wobei bevorzugt an einem ersten Ende des Objekts OB der Abtastvorgang startet und über die Oberfläche des Objekts OB zu einem gegenüber liegenden zweiten Ende geführt wird. Die Geschwindigkeit der Schwenkbewegung um die Drehachse DA kann dabei so eingestellt werden, dass die gewünschte Auflösung erreicht wird. Typischerweise erhöht sich die Auflösung der Laserscanvorrichtung VO mit zunehmender Abtastrate des Lichtsensors LS, wobei jedoch zur Bewältigung der damit größeren Datenmenge eine geringere Geschwindigkeit der Schwenkbewegung um die Drehachse DA vorgegeben wird.

Die mit einer derartigen Laserscanvorrichtung VO erreichbare Auflösung beträgt bei einer typischen Länge des Objekts von einigen Metern, wie sie üblicherweise bei der Vermessung von Schnittholz vorliegt, weniger als 0,1 mm. Durch die variable Geschwindigkeit der Schwenkbewegung erhält man somit eine justierbare Auflösung.

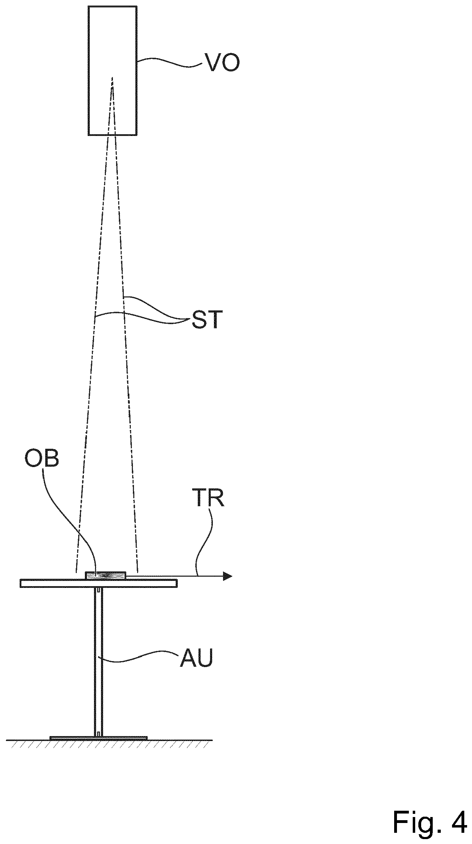

In Fig. 4 ist die Laserscanvorrichtung VO aus Fig. 1 nochmals in einer Seitenansicht gezeigt, wobei die Blickrichtung einer Längsachse des Objekts OB folgt. Typischerweise wird das Objekt OB entlang einer Transportrichtung TR im Quertransport unter der Laserscanvorrichtung VO durchgeführt. Dabei ist mittels der Laserscanvorrichtung VO auch eine Vermessung von gleichzeitig zwei Seiten des Objekts OB möglich, in dem das Objekt OB entweder schräg zur Laserscanvorrichtung VO angeordnet ist oder ein Schrägstellen des Objekts OB erfolgt. Demnach kann beispielsweise ein Brett gleichzeitig an seiner Hauptfläche und seiner schmäleren Seitenfläche vermessen werden.

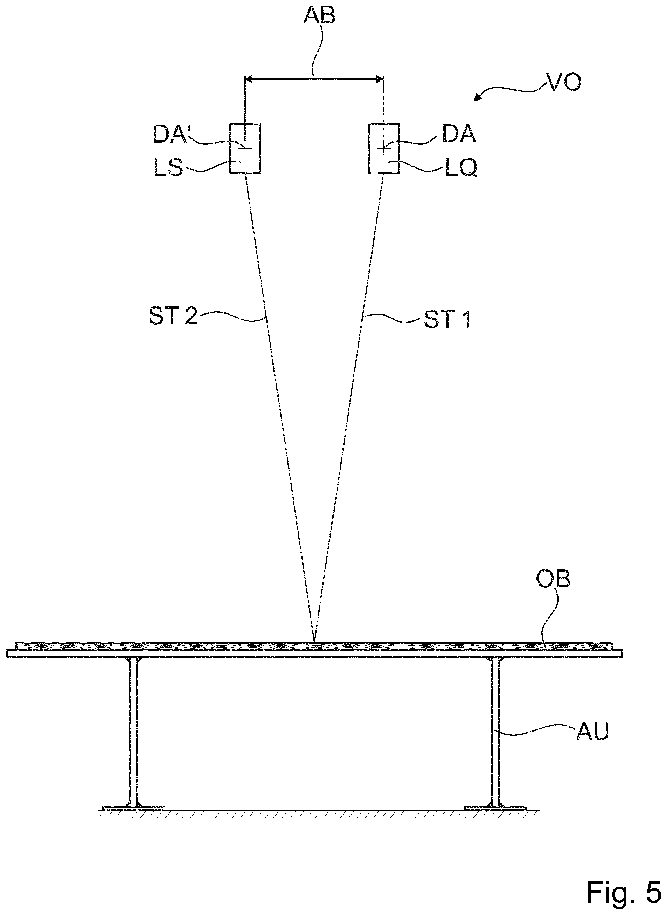

Unter Bezugnahme auf Fig. 5 wird nachfolgend eine weitere Ausführungsform der erfindungsgemäßen Laserscanvorrichtung VO gezeigt. Diese Darstellung folgt dem in Fig. 1 gezeigten und zur Vermeidung von Wiederholungen werden nachfolgend lediglich die Unterschiede zur Ausführungsform gemäß Fig. 1 erläutert.

Diese Laserscanvorrichtung VO weist anstelle des Schwenkarms die um die Drehachse DA schwenkbare Lichtquelle LQ und den um die Drehachse DA' schwenkbaren Lichtsensor LS auf. Der Lichtsensor LS und die Lichtquelle LQ sind dabei bezüglich der Drehachsen DA und DA' nebeneinanderliegend angeordnet. Bei einer horizontalen Ausrichtung von Lichtsensor LS und Lichtquelle LQ sind diese bevorzugt entlang einer gedachten Linie angeordnet. Es sind aber auch andere Anordnungen denkbar, bei denen beispielsweise Lichtsensor LS und Lichtquelle LQ in vertikaler Richtung einen Versatz aufweisen. Durch eine korrelierte Drehung um die Drehachsen DA und DA' in dergleichen Umlaufrichtung mittels Schrittmotoren kann nun wiederum eine Triangulationsmessung erfolgen, wobei in dieser Ausführungsform Lichtsensor LS und Lichtquelle LQ jeweils mit einem Winkelmesser versehen sein können, die ihre gemessenen Drehwinkel zur Weiterverarbeitung bereitstellen. Der Abstand AB von Lichtsensor LS und Lichtquelle LQ kann variabel festlegbar sein, so dass sich je nach gewünschten Anforderungen beispielsweise die Tiefenschärfe der Laserscanvorrichtung VO durch einen anderen Abstand AB beeinflussen lässt.

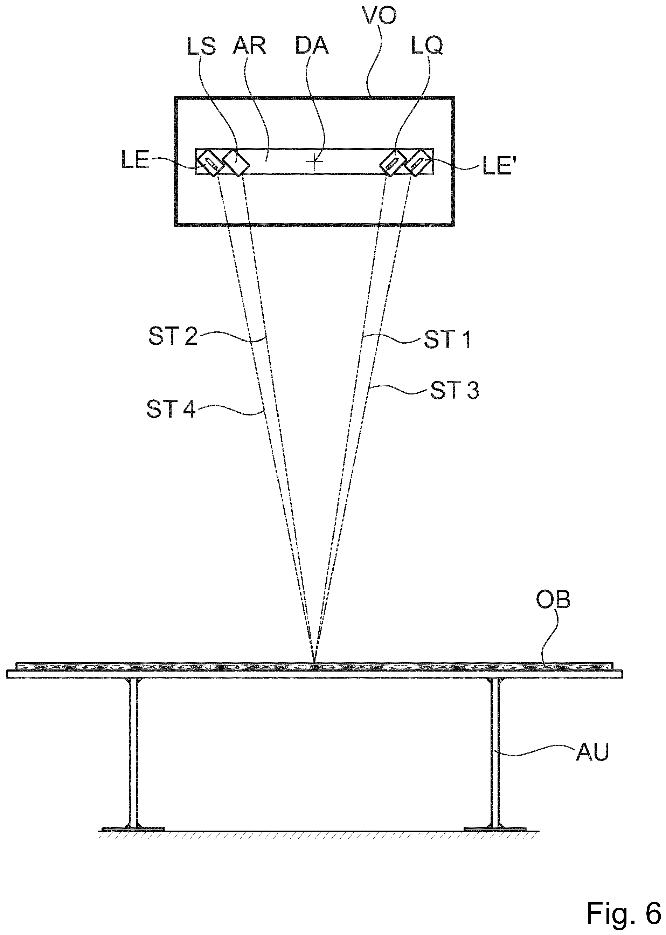

Eine weitere Ausführungsform der erfindungsgemäßen Laserscanvorrichtung VO wird nachfolgend unter Bezugnahme auf Fig. 6 gezeigt. Diese Darstellung folgt wiederum dem in Fig. 1 gezeigten und es werden nachfolgend lediglich die Unterschiede zur Ausführungsform gemäß Fig. 1 erläutert.

Diese Laserscanvorrichtung VO weist an gegenüberliegenden Enden des Schwenkarms AR jeweils zusätzliche lichtemittierende Strahler LE und LE' auf, benachbart zur Lichtquelle LQ und zum Lichtsensor LS angebracht sind. Die beiden Strahler LE und LE' leuchten das Objekt OB farbig aus, wobei sich durch die Aufteilung auf die beiden Strahler LE und LE' eine diffuse Ausleuchtung ergibt, die wenig Glanzstellen aufweist. Das Ausleuchten des Objekts OB mittels der beiden lichtemittierenden Strahler LE und LE' ist in Fig. 6 durch die Strahlengänge ST3 und ST4 angedeutet.

In manchen Anwendungsfällen kann es jedoch ausrechend sein, nur einen lichtemittierenden Strahler vorzusehen, der entweder benachbart zur Lichtquelle LQ oder zum Lichtsensor LS angebracht sein kann. Die farbige Ausleuchtung kann zur optischen Qualitätsbeurteilung der Oberfläche des Objekts herangezogen werden. In wiederum anderen Anwendungsfällen kann alternativ oder zusätzlich entweder benachbart zur Lichtquelle LQ oder zum Lichtsensor LS ein Punktlaser als zusätzlicher oder weiterer zusätzlicher lichtemittierender Strahler angebracht sein. Mittels eines Punktlasers lassen sich Faserungen oder Astlöcher optisch erkennen, so dass auch eine Beurteilung bezüglich Festigkeiten oder dergleichen möglich ist.

Die in Fig. 6 beschriebene Ausführungsform lässt sich auch auf die in Fig. 5 gezeigte Ausführungsform mit zwei Drehachsen übertragen. Es wäre auch denkbar, eine von der oder den Drehachsen unabhängige Befestigung für den oder die lichtemittierende Strahler vorzusehen.

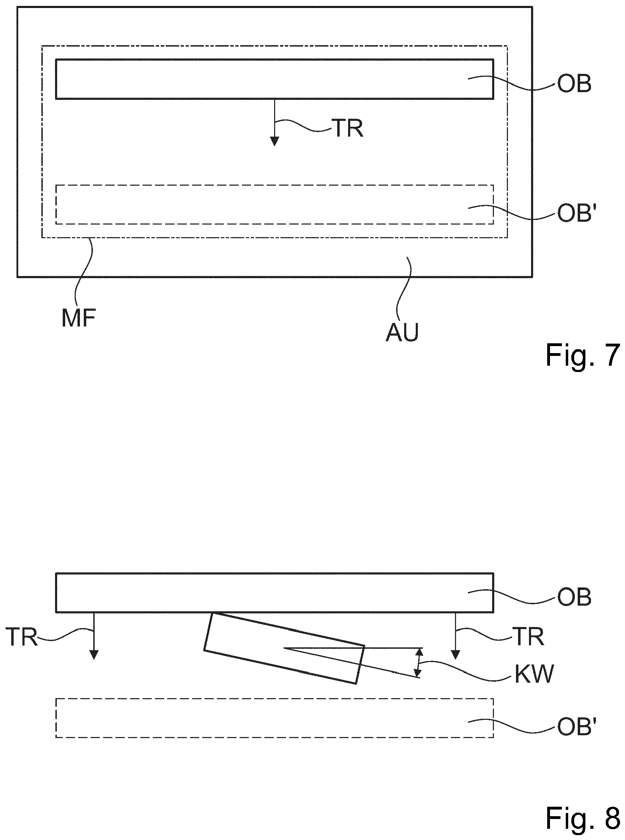

Die erfindungsgemäßen Laserscanvorrichtungen VO gemäß den Figuren 1 bis 6 sind in der Lage, Objekte OB optisch zu vermessen, die sich entweder im Stillstand oder im Durchlauf befinden. Diese beiden Varianten werden nachfolgend unter Bezugnahme auf die Fig. 7 bzw. auf die Fig. 8 nochmals genauer erläutert.

In Fig. 7 ist eine Draufsicht auf die Aufnahme AU beim Vermessen des Objekts OB mit der Laserscanvorrichtung VO gezeigt. Bei der optischen Vermessung im Stillstand ist es ausreichend, dass das Messfenster MF, das durch die Größe eines Belichtungsfelds der Lichtquelle LQ bzw. des Sichtfelds des Lichtsensors LS gegeben ist, in etwa den Abmessungen des Objekts OB entspricht. Falls die Laserscanvorrichtung VO jedoch im Durchlauf betrieben wird, bewegt sich das Objekt OB mit der Vorlaufgeschwindigkeit in Richtung der Transportrichtung TR, während die Laserscanvorrichtung VO die Schwenkbewegung um die Drehachse DA ausführt.

Demnach kann für eine vollständige Messung der Oberfläche des Objekts OB, das beispielsweise wie in Fig. 7 gezeigt, während der Schwenkbewegung die Position des gestrichelt eingezeichneten Objekts OB' einnimmt, mit einem derart großen Messfenster MF versehen sein, dass sowohl das Objekt OB an seiner ursprünglichen Stelle als auch an der Position OB' im Messfenster zu liegen kommt.

In Fig. 8 ist eine weitere Ausführungsform für eine Laserscanvorrichtung VO im Durchlaufbetrieb gezeigt. Hier wird die Laserscanvorrichtung VO um einen Kippwinkel KW einer Stellachse senkrecht zur Oberfläche des Objekts OB verkippt, so dass zu Beginn der Messung der in Fig. 8 am linken Blattrand liegende Teil des Objekts OB bei vollständig geschwenkter Position des Schwenkarms AR abgetastet wird.

Während der Bewegung des Objekts OB in Richtung der Transportrichtung TR erfolgt ein kontinuierliches Schwenken des Schwenkarms AR in Richtung des rechten Endes des Objekts OB, wobei aufgrund des Verkippens um den Kippwinkel KW die Lichtquelle bei vollständig nach rechts verschwenkten Schwenkarm das rechte Ende des Objekts OB' abtasten kann. Der Kippwinkel KW ist dabei an die Transportgeschwindigkeit des Objekts OB auf der Auflage AU angepasst. Nachdem die Laserscanvorrichtung VO die in Fig. 8 gezeigte Endposition am Objekt OB' erreicht hat, erfolgt ein schnelles Zurückschwenken, um die Messung für das nächste Objekt OB wieder am linken Ende beginnen zu lassen.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar.

## Patentansprüche

1. Laserscanvorrichtung zur optischen Qualitätsbeurteilung und Vermessung von Objekten (OB) im Quertransport, insbesondere von Schnitthölzern, wobei die Laserscanvorrichtung (VO) zur Triangulationsmessung eine Lichtquelle (LQ) zur Abgabe eines Laserstrahls (ST1) in Richtung des Objekts (OB) und einen Lichtsensor (LS) zur Aufnahme der von dem Objekt (OB) reflektierten Laserstrahl (ST2) umfasst, **dadurch gekennzeichnet, dass** die Lichtquelle (LQ) und der Lichtsensor (LS) relativ zu dem Objekt (OB) korreliert und in gleicher Drehrichtung schwenkbar sind, so dass die Lichtquelle (LQ) zusammen mit dem Lichtsensor (LS) das Objekt (OB) entlang einer Längsachse des Objekts (OB) abscannt, um ein drei-dimensionales Abbild der Oberfläche des Objekts (OB) zu erzeugen.

2. Laserscanvorrichtung nach Anspruch 1, bei der die Lichtquelle (LQ) und der Lichtsensor (LS) jeweils um eine Drehachse (DA; DA') schwenkbar sind oder bei der die Lichtquelle (LQ) und der Lichtsensor (LS) auf gegenüberliegenden Enden eines gemeinsamen Schwenkarms (AR) montiert sind, der um eine Drehachse (DA) schwenkbar ist.

3. Laserscanvorrichtung nach einem der Ansprüche 1 bis 2, bei der die Lichtquelle (LQ) eine Laserdiode ist und/oder der Lichtsensor (LS) eine CCD-Kamera ist.

4. Laserscanvorrichtung nach einem der Ansprüche 1 bis 3, bei der die von dem Lichtsensor (LS) abgegebenen Signale zur Erzeugung des dreidimensionalen Abbilds der Oberfläche des Objekts (OB) während des Schwenkens mit einem jeweiligen Drehwinkel (DW) korreliert sind.

5. Laserscanvorrichtung nach einem der Ansprüche 1 bis 4, bei der das Schwenken mit einer einstellbaren Geschwindigkeit erfolgt.

6. Laserscanvorrichtung nach einem der Ansprüche 1 bis 5, bei der die Intensität des von der Lichtquelle (LQ) abgegebenen Lichtstrahls (ST1) in Abhängigkeit des Drehwinkels (DW) gesteuert ist.

7. Laserscanvorrichtung nach einem der Ansprüche 1 bis 6, bei der eine Empfindlichkeit des Lichtsensors (LS) von der Drehposition in Abhängigkeit des Drehwinkels (DW) parametrisiert ist.

8. Laserscanvorrichtung nach einem der Ansprüche 1 bis 7, bei der die optische Vermessung des Objekts (OB) im Stillstand erfolgt.

9. Laserscanvorrichtung nach einem der Ansprüche 1 bis 8, bei der die optische Vermessung des Objekts (OB) im Durchlauf erfolgt.

10. Laserscanvorrichtung nach Anspruch 9, bei der das Sichtfeld des Lichtsensors (LS) und/oder die Beleuchtungsfläche der Lichtquelle (LQ) an die Durchlaufgeschwindigkeit des Objekts (OB) angepasst sind.

11. Laserscanvorrichtung nach Anspruch 9, bei der eine Anpassung an die Durchlaufgeschwindigkeit durch eine Verkippung (KW) um eine senkrecht zur Drehachse (DA; DA') und der Durchlaufrichtung (TR) angeordnete Stellachse erfolgt, wobei die Verkippung (KW) an die Durchlaufgeschwindigkeit angepasst ist.

12. Laserscanvorrichtung nach Anspruch 11, bei der das Schwenken von einem ersten Ende zu einem zweiten Ende des Objekts hin erfolgt, wobei bei Erreichen des zweiten Endes ein schnelles Zurückschwenken erfolgt.

13. Laserscanvorrichtung nach einem der Ansprüche 1 bis 12, bei der die optische Vermessung des Objekts (OB) auf einer Auflage erfolgt, die eine Kippeinrichtung des Objekts (OB) aufweist, um mehrere Seitenflächen des Objekts (OB) vermessen zu können.

14. Laserscanvorrichtung nach einem der Ansprüche 1 bis 13, bei der zusätzliche lichtemittierende Strahler, vorzugsweise benachbart zur Lichtquelle (LQ) oder benachbart zum Lichtsensor (LS), angebracht sind, die das Objekt farbig oder punktförmig ausleuchten.

15. Laserscanvorrichtung nach einem der Ansprüche 1 bis 14, bei der der Lichtsensor (LS) aus einer Mehrfachanordnung von lichtempfindlichen Elementen gebildet ist, wobei ein für eine Auswertung relevanter Bereich der Mehrfachanordnung in Abhängigkeit der Schwenkbewegung gewählt ist.
